(19) Europäisches Patentamt / European Patent Office / Office européen des brevets

(11) **EP 3 572 397 B1**

(12) **EUROPEAN PATENT SPECIFICATION**

(45) Date of publication and mention
of the grant of the patent:
**01.09.2021 Bulletin 2021/35**

(21) Application number: **18741885.0**

(22) Date of filing: **18.01.2018**

(51) Int Cl.:
***C07C 231/08*** (2006.01)       ***C07C 231/24*** (2006.01)
***C07C 231/12*** (2006.01)       ***C07C 233/18*** (2006.01)
***C07C 233/03*** (2006.01)

(86) International application number:
**PCT/JP2018/001367**

(87) International publication number:
**WO 2018/135574 (26.07.2018 Gazette 2018/30)**

(54) **METHOD FOR PRODUCING N-(ALPHA-HYDROXYETHYL)FORMAMIDE AND METHOD FOR PRODUCING N-VINYLFORMAMIDE**

VERFAHREN ZUR HERSTELLUNG VON N-(ALPHA-HYDROXYETHYL)FORMAMID UND VERFAHREN ZUR HERSTELLUNG VON N-VINYLFORMAMID

PROCÉDÉ DE PRODUCTION DE N-(ALPHA-HYDROXYÉTHYL)FORMAMIDE ET PROCÉDÉ DE PRODUCTION DE N-VINYLFORMAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.01.2017 JP 2017006763**

(43) Date of publication of application:
**27.11.2019 Bulletin 2019/48**

(73) Proprietor: **Mitsubishi Chemical Corporation**
**Tokyo 100-8251 (JP)**

(72) Inventors:
• **SOMEYA Yuuichi**
**Tokyo 100-8251 (JP)**
• **TANAKA Akira**
**Tokyo 100-8251 (JP)**
• **MORI Yasuharu**
**Tokyo 100-8251 (JP)**

(74) Representative: **Hoffmann Eitle**
**Patent- und Rechtsanwälte PartmbB**
**Arabellastraße 30**
**81925 München (DE)**

(56) References cited:
WO-A1-2008/120769     JP-A- S6 259 248
JP-A- S6 314 761       JP-A- H06 184 071
JP-A- H06 298 713      JP-A- S60 149 551
JP-A- S60 193 953      JP-A- S61 286 356
JP-A- S62 195 352      JP-A- 2000 191 625
JP-A- 2006 089 419     JP-A- 2007 023 284

**Description**

TECHNICAL FIELD

The present invention relates to a method for producing N-($\alpha$-hydroxyethyl)formamide and a method for producing N-vinylformamide.

This application is based upon and claims the benefit of priority of the prior Japanese Patent Application No. 2017-006763 filed in Japan on January 18, 2017.

BACKGROUND ART

N-($\alpha$-hydroxyethyl)formamide is an important substance as an intermediate raw material for N-vinylformamide.

N-($\alpha$-hydroxyethyl)formamide is obtained by, for example, reacting (hydroxylation reaction) formamide with acetaldehyde in the presence of a basic catalyst such as potassium hydrogencarbonate and filtering a slurry-like reaction product (See Patent Document 1). Patent Document 2 relates to a method of producing a guanidine derivative. Patent Document 3 is directed to a method of preparing a metal compound of an azo compound using pump circulation.

CITATION LIST

PATENT DOCUMENT

   Patent Document 1: JP 6-298713 A
   Patent Document 2: JP 2000-191625 A
   Patent Document 3: JP 2007-023284 A

SUMMARY OF THE INVENTION

PROBLEM TO BE SOLVED BY THE INVENTION

However, in the conventional method for producing N-($\alpha$-hydroxyethyl)formamide, the conditions under which the reaction is conducted while stably forming a slurry are not known and the yield (hydroxylation reaction yield) of N-(a-hydroxyethyl)formamide is not necessarily satisfied.

In the hydroxylation reaction, N-($\alpha$-hydroxyethyl)formamide is formed in a reaction solution as well but this reaction is an equilibrium reaction. Hence, the hydroxylation reaction is not completed unless there is a movement of equilibrium due to the precipitation of N-($\alpha$-hydroxyethyl)formamide in the reaction solution. Hence, it is required to sufficiently increase the reaction rate of the hydroxylation reaction in order to increase the reaction yield of the hydroxylation reaction.

As a means for increasing the reaction rate of the hydroxylation reaction, a method is conceivable in which the hydroxylation reaction is conducted by increasing the amount of catalyst used, but an excessive amount of catalyst causes a side reaction to condense the aldehyde and further the catalyst also reacts with formamide to be time-dependently degenerated and to exhibit decreased activity, and it is thus preferable to use the catalyst in the minimum amount necessary in a short time.

Furthermore, poor filtration that it takes time to filter the reaction product may be caused in the case of conducting filtration in the next step when the crystals of N-($\alpha$-hydroxyethyl)formamide are too small even if N-($\alpha$-hydroxyethyl)formamide is precipitated. On the other hand, the crystals may adhere to the reactor and pipe used in the reaction and clogging may be caused when the crystals of N-($\alpha$-hydroxyethyl)formamide are too large. In addition, it is difficult to send the slurry by the slurry pump upon filtration in some cases. Furthermore, the raw materials remain inside the coarse crystals and the percent conversion does not increase.

Hence, it is required to precipitate N-($\alpha$-hydroxyethyl)formamide from the solvent in a proper size in order to obtain a slurry of N-($\alpha$-hydroxyethyl)formamide which exhibits excellent handling properties such as filtration property and liquid sending property while suppressing clogging of the reactor and pipe.

However, in the conventional method for producing N-($\alpha$-hydroxyethyl)formamide, the conditions for precipitating N-($\alpha$-hydroxyethyl)formamide in a proper size are not known.

The invention has been made in view of the above circumstances, and an object thereof is to provide a method for producing N-($\alpha$-hydroxyethyl)formamide by which N-($\alpha$-hydroxyethyl)formamide can be produced at a high yield and a method for producing N-vinylformamide.

MEANS FOR SOLVING PROBLEM

The invention is defined by the appended claims and concerns a method for producing N-($\alpha$-hydroxyethyl)formamide, including:

reacting formamide with acetaldehyde in a solvent in presence of a basic catalyst in a reaction tank;
precipitating N-($\alpha$-hydroxyethyl)formamide obtained as a crystal in the reaction tank; and
recovering the crystal of N-($\alpha$-hydroxyethyl)formamide precipitated from the reaction tank, in which
an n+1-th reaction is conducted in a state in which the reaction solution is discharged from the reaction tank and at least a part of the crystals of N-($\alpha$-hydroxyethyl)formamide obtained by an n-th reaction remains in the reaction tank (where n is a natural number) when the production of N-($\alpha$-hydroxyethyl)formamide is conducted plural times in the same reaction tank.

EFFECT OF THE INVENTION

According to the method for producing N-($\alpha$-hydroxyethyl)formamide of the invention, it is possible to produce N-($\alpha$-hydroxyethyl)formamide at a high yield. Moreover, it is possible to cut down the labor costs of washing and the cost for treating the waste washwater. In addition, it is possible to cut down the cost of equipment and labor costs for adding the seed crystal.

According to the method for producing N-vinylformamide of the invention, it is possible to produce N-vinylformamide at a high yield. Moreover, it is possible to cut down the labor costs of washing and the cost for treating the waste washwater. In addition, it is possible to cut down the cost of equipment and labor costs for adding the seed crystal.

BRIEF DESCRIPTION OF DRAWINGS

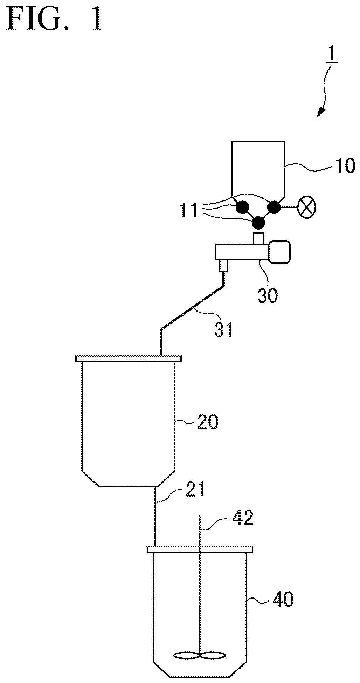

Fig. 1 is a schematic configuration diagram illustrating an apparatus for producing N-($\alpha$-hydroxyethyl)formamide used in Examples and Comparative Examples.

MODE(S) FOR CARRYING OUT THE INVENTION

<<Method for producing N-($\alpha$-hydroxyethyl)formamide>>

The method for producing N-($\alpha$-hydroxyethyl)formamide of the invention is a method for producing N-($\alpha$-hydroxyethyl)formamide, which includes reacting formamide with acetaldehyde in a solvent in presence of a basic catalyst in a reaction tank, precipitating N-($\alpha$-hydroxyethyl)formamide obtained as a crystal in the reaction tank, and recovering the crystal of N-($\alpha$-hydroxyethyl)formamide precipitated from the reaction tank and in which an n+1-th reaction is conducted in a state in which the reaction solution is discharged from the reaction tank and at least a part of the crystals of N-($\alpha$-hydroxyethyl)formamide obtained by an n-th reaction remains in the reaction tank (where n is a natural number) when the production of N-($\alpha$-hydroxyethyl)formamide is conducted plural times in the same reaction tank.

The amount of crystals present in the reaction tank at the time of n+1-th reaction is preferably from 10 g to 200 kg or less, more preferably from 50 g to 150 kg, still more preferably from 100 g to 100 kg, and particularly preferably from 200 g to 50 kg per 1 $m^3$ of the inner volume of the reaction tank.

The crystallization speed is not too slow and the crystals are likely to be dispersed in the liquid when the amount of crystals present in the reaction tank is equal to or more than the lower limit value.

The amount of seed crystals in the reaction tank is not too much and a decrease in production amount per one batch is suppressed when the amount of crystals present in the reaction tank is equal to or less than the upper limit value.

The method for producing N-($\alpha$-hydroxyethyl)formamide of the invention includes the following hydroxylation reaction step.

[Hydroxylation reaction step]

This step is a step of obtaining N-($\alpha$-hydroxyethyl)formamide by reacting (hydroxylation reaction) formamide with acetaldehyde in a solvent insoluble in water in the presence of a basic catalyst.

The molar ratio of formamide to acetaldehyde (formamide : acetaldehyde) is preferably from 1 : 1 to 1 : 10 and more preferably from 1 : 1 to 1 : 5. It is possible to increase the percent conversion of formamide by setting the molar ratio of acetaldehyde to be excessive. Incidentally, it is not preferable that unreacted formamide is excessively present in the reaction system since the crystals of N-($\alpha$-hydroxyethyl)formamide formed are dissolved.

The reaction temperature in the hydroxylation reaction can be measured by using a thermometer to be usually industrially used such as a thermocouple thermometer. The reaction temperature is usually about from -10°C to 100°C. However, it is preferable to set the temperature at the time of the reaction to be as low as possible in order to prevent a decrease in the activity of catalyst and the side reactions, and the reaction temperature is more preferably from 0°C to 40°C from the viewpoint of the hydroxylation reaction yield from formamide to N-(α-hydroxyethyl)formamide and of crystallizing the N-(α-hydroxyethyl)formamide. Incidentally, the method for adjusting the reaction temperature is not particularly limited, but for example, there is a method in which the reactor is equipped with a jacket and the reaction temperature is adjusted while circulating cooling water in the jacket.

The hydroxylation reaction for forming N-(α-hydroxyethyl)formamide by reacting formamide with acetaldehyde and the precipitation reaction for crystallizing the N-(α-hydroxyethyl)formamide formed are both an exothermic reaction, and it is required to detect the heat value by reaction in order to confirm whether these reactions are sufficiently proceeding.

The total heat value by these reactions is not particularly limited, but for example, it can be detected in the flowing manner.

The total heat value is detected by measuring the temperature before the inflow of cooling water to be supplied to the jacket of the reactor, the temperature at the time of outflow, and the flow rate of the cooling water which has actually flowed into the jacket and calculating the total heat value by the following Equation (I) using these values.

$$\text{Total heat value} = \text{temperature difference before and after flow} \times \text{flow rate of}$$

$$\text{cooling water} \times \text{specific heat of cooling water} \cdots \text{(I)}$$

As a solvent used in the hydroxylation reaction, a solvent insoluble in water is used. More specifically, it is a solvent having a property to dissolve 1 g or less of water in 100 g thereof at 25°C. As such a solvent, aliphatic hydrocarbons such as hexane, heptane, and cyclohexane; aromatic hydrocarbons such as benzene, toluene, and xylene; and halogenated hydrocarbons such as methylene chloride, and chloroform; and the like are preferable from the viewpoint of crystallizing N-(α-hydroxyethyl)formamide formed.

The amount of the solvent used is preferably from 0.2 to 10 times the mass of formamide.

The basic catalyst to be used in the hydroxylation reaction is not particularly limited as long as it is a general basic compound, but it is preferably a weak basic salt composed of a strong base and a weak acid having a pKa value of from 4 to 15, and specific examples thereof may include sodium carbonate, sodium hydrogencarbonate, potassium carbonate, potassium hydrogencarbonate, lithium carbonate, lithium hydrogencarbonate, potassium phosphate, potassium monohydrogen phosphate, and sodium pyrophosphate. Among these, potassium hydrogencarbonate is preferable from the viewpoint of being able to decrease by-products (for example, aldol condensate of acetaldehyde) to be formed at the time of the reaction of formamide with acetaldehyde.

The amount of the basic catalyst used is preferably from 0.01% to 10% by moles and more preferably from 0.1% to 2% by moles with respect to formamide.

By precipitating the crystals of N-(α-hydroxyethyl)formamide, most of the solvent can be separated and recovered by simply collecting the crystals by a method such as filtration.

The method for filtering the slurry is not particularly specified, the slurry may be subjected to gravity filtration or the slurry may be supplied to the filter by using a slurry pump while controlling the liquid volume of the slurry by using a supply control valve, and the filter also may be any method such as filter filtration or centrifugal filtration.

The reaction mixture contains unreacted formamide and acetaldehyde, an aldol condensate of acetaldehyde which is a by-product of the hydroxylation reaction, the basic catalyst, the reaction solvent, and the like in addition to N-(α-hydroxyethyl)formamide which is a reaction product of formamide with acetaldehyde.

As a specific method for conducting the hydroxylation reaction, there is a method in which the reaction tank in which N-(α-hydroxyethyl)formamide has been produced is not washed or only a part thereof is washed and the production of N-(α-hydroxyethyl)formamide is conducted again in this reaction tank.

The crystals of N-(α-hydroxyethyl)formamide which are present in advance act as a seed crystal from the time point at which the hydroxylation reaction starts and smoothly advance the precipitation of N-(α-hydroxyethyl)formamide, and it is thus important to start the hydroxylation reaction in the presence of the crystals.

In this manner, when crystals of N-(α-hydroxyethyl)formamide are present in the reaction solution from the time point at which the hydroxylation reaction starts, the precipitation of N-(α-hydroxyethyl)formamide begins as the hydroxylation reaction starts and the reaction rate of the hydroxylation reaction can be increased.

In addition, the equilibrium of the hydroxylation reaction moves in the direction of the N-(α-hydroxyethyl)formamide forming reaction by precipitation, and thus great heat generation by reaction is detected when the precipitation of N-(α-hydroxyethyl)formamide occurs in the middle of the hydroxylation reaction. It is possible to confirm whether the hydroxylation reaction is sufficiently proceeding or not by detecting this heat generation by reaction.

Furthermore, the basic solvent used in the hydroxylation reaction is occluded in the crystals along with the precipitation of N-(α-hydroxyethyl)formamide. Hence, it is preferable that the precipitation of N-(α-hydroxyethyl)formamide begins from the time point at which the hydroxylation reaction starts since the separation of basic catalyst from the reaction solution phase also begins and side reactions such as the polymerization reaction of aldehyde can be suppressed.

The method for producing N-(α-hydroxyethyl)formamide of the invention is a batch type in which the hydroxylation reaction is discontinuously conducted.

In the case of a batch type, the entire amounts of the reaction raw materials (formamide, acetaldehyde, basic catalyst, and reaction solvent) are charged into the reaction tank at once or a part of the reaction raw materials are charged into the reaction tank in advance and the remainders of reaction raw materials are continuously or intermittently supplied thereto to conduct the hydroxylation reaction, and the reaction solution is discharged from the reaction tank when the hydroxylation reaction is completed.

In the case of a batch type, crystals of N-(α-hydroxyethyl)formamide remain in the reaction tank in which the reaction has been conducted when the hydroxylation reaction is stopped and then the reaction solution is discharged from the reaction tank. In this case, the hydroxylation reaction is restarted in the presence of crystals of N-(α-hydroxyethyl)formamide formed by the hydroxylation reaction before stopping. In other words, the remaining crystals of N-(α-hydroxyethyl)formamide are reused by discharging the reaction solution after the hydroxylation reaction is stopped and using the reaction tank again as a hydroxylation reaction tank without washing the interior thereof.

It is special to conduct the reaction while the crystals remain. In general, the crystals grow when conducting the reaction while the crystals remain. However, in the present reaction, the crystals do not grow under proper stirring but are dispersed in the reaction liquid to be a seed crystal.

In the invention, a solvent insoluble in water and acetaldehyde may be spread in the reaction tank from which the reaction solution has been discharged after the hydroxylation reaction is stopped and the dropwise addition of formamide in which the basic catalyst is dissolved thereonto may be started to restart the hydroxylation reaction or a mixed liquid of a solvent insoluble in water and acetaldehyde and formamide in which the basic catalyst is dissolved may be simultaneously supplied to the reaction tank to restart the reaction.

The crystals containing N-(α-hydroxyethyl)formamide formed by the hydroxylation reaction before stopping act as a seed crystal from the time point at which the hydroxylation reaction restarts and smoothly advance the precipitation of N-(α-hydroxyethyl)formamide in the hydroxylation reaction restarted as well, and it is thus important to restart the hydroxylation reaction in the presence of the crystals.

N-(α-hydroxyethyl)formamide dissolves in acetaldehyde, the crystals is thus required to remain when a mixed liquid of acetaldehyde and a water-insoluble solvent is supplied in order to act as a seed crystal. For that purpose, it is required that the crystals remain at the portion which is not immersed in the liquid or the crystals remain in the liquid in an amount exceeding the solubility. The latter method may be adopted in a semi-batch reaction, but the size of reaction kettle increases accordingly.

In this manner, when the crystals of N-(α-hydroxyethyl)formamide are present in the reaction solution from the time point at which the hydroxylation reaction restarts, the precipitation of N-(α-hydroxyethyl)formamide begins as the hydroxylation reaction starts and the reaction rate of the hydroxylation reaction to restart can be increased.

In addition, the equilibrium of the hydroxylation reaction moves in the direction of the N-(α-hydroxyethyl)formamide forming reaction by precipitation, and thus great heat generation by reaction is detected when the precipitation of N-(a-hydroxyethyl)formamide occurs in the middle of the hydroxylation reaction.

The basic solvent used in the hydroxylation reaction is occluded in the crystals along with the precipitation of N-(α-hydroxyethyl)formamide. Hence, it is preferable that the precipitation of N-(α-hydroxyethyl)formamide begins from the time point at which the hydroxylation reaction restarts since the separation of basic catalyst from the reaction solution phase also begins and side reactions such as the polymerization reaction of aldehyde can be suppressed.

The hydroxylation reaction is conducted while stirring the reaction solution under the condition that the stirring power per unit volume of the reactant is in a range of from 0.1 to 10.0 $kW/m^3$. The crystals stuck on the wall are released by stirring and fall into the reaction liquid, thus the crystals are dispersed in the reaction liquid and become a seed crystal in the next reaction, and a clean slurry is formed without adding a new seed crystal on purpose.

The stirring power is preferably from 0.4 to 6.0 $kW/m^3$ and more preferably from 1.0 to 3.0 $kW/m^3$. The seed crystals are neatly dispersed in the reaction liquid when the stirring power is 0.1 $kW/m^3$ or more, and thus the crystals can be easily collected without solidifying at the lower portion of the reaction tank. In addition, the crystals of N-(α-hydroxyethyl)formamide to be precipitated from the solvent do not grow too large and the reaction slurry can be easily sent by the slurry pump. In addition, the crystals of N-(α-hydroxyethyl)formamide hardly adhere to the reactor and pipe and clogging of the reactor and pipe can be suppressed.

On the other hand, when the stirring power is 10.0 $kW/m^3$ or less, destruction of the crystals of N-(α-hydroxyethyl)formamide and poor growth of the crystals hardly occur and the crystals of N-(α-hydroxyethyl)formamide do not grow too small. Hence, a reaction slurry exhibiting favorable filtration property can be obtained and the reaction slurry can be filtered in a short time.

It is preferable that the hydroxylation reaction is conducted at a stirring power of from 0.1 to 10.0 kW/m$^3$ for 2/3 or more of the total reaction time and it is more preferable the entire hydroxylation reaction is conducted at a stirring power of from 0.1 to 10.0 kW/m$^3$.

Here, the term "stirring power" means the electric power consumed by an electric machine (motor) for stirring rotation. The stirring power can be determined by subtracting the load of bearing (electric power consumption when there is no reactant) from the electric power consumption at the time of operation and dividing the resultant by the volume (m$^3$) of the reactant. In addition, the stirrer may be equipped with a stirring dynamometer, a torque meter, or the like and the torque difference between the reaction time and the empty time (in a case in which there is no reactant) may be directly measured.

The average particle diameter of the crystals of N-($\alpha$-hydroxyethyl)formamide is preferably from 0.3 to 2.0 mm, more preferably from 0.5 to 2.0 mm, still more preferably from 0.8 to 1.2 mm, and particularly preferably from 0.9 to 1.1 mm. The filtration property when filtering the reaction slurry is improved when the average particle diameter of the crystals of N-($\alpha$-hydroxyethyl)formamide is 0.3 mm or more. On the other hand, the reaction slurry can be easily sent by the slurry pump when the average particle diameter of the crystals of N-($\alpha$-hydroxyethyl)formamide is 2.0 mm or less. In addition, the crystals of N-($\alpha$-hydroxyethyl)formamide hardly adhere to the reactor and pipe and clogging of the reactor and pipe can be suppressed.

The measurement of the average particle diameter of the crystals of N-(a-hydroxyethyl)formamide may be any of measurement of particle size distribution using a sieve, measurement by microscopic observation, sedimentation method, or the like, but microscopic observation is simple when the actual particle diameter, the stability of particles, and the like are taken into consideration.

In the measurement by microscopic observation, one subjected to image processing may be measured or the image may be directly measured. For example, the average value of the diameters of the equivalent circles having equivalent areas of the crystals when about 10 to 20 crystals of N-($\alpha$-hydroxyethyl)formamide which are arbitrarily sampled are observed in planar view under an optical microscope is regarded as the average particle diameter of the crystals of N-($\alpha$-hydroxyethyl)formamide. In a case in which the particles are close to a spherical shape, the average value of the major axis and the minor axis may be determined and taken as the average particle diameter of the crystals of N-($\alpha$-hydroxyethyl)formamide.

As the reaction tank in the invention, it is preferable to use a reaction tank of which the interior is hardly corroded by the reaction solution from the viewpoint of maintenance. Examples of such a reaction tank may include a reaction tank made of stainless steel, but the reaction tank is not limited thereto.

In addition, as the reaction tank in the invention, it is preferable to use a dedicated reaction tank for conducting only the hydroxylation reaction, but the reaction tank is not limited thereto.

[Effect]

According to the method for producing N-($\alpha$-hydroxyethyl)formamide of the invention, there is no quality problem even when the reaction product remains in the reaction system, the crystals remaining in the reactor are dispersed in the reaction liquid to be a seed crystal, and the product of the next reaction is precipitated without newly adding a seed crystal. Hence, it is possible to cut down the labor costs for washing the kettle, the cost for treating the waste washwater, and the cost of equipment and labor costs for adding the seed crystal. In addition, it is possible to produce N-(a-hydroxyethyl)formamide at a high yield.

The N-($\alpha$-hydroxyethyl)formamide obtained by the invention can be used in the production of N-($\alpha$-alkoxyethyl)formamide by being reacted with an alcohol in the presence of an acid catalyst and can be thus used as an intermediate raw material for N-vinylformamide.

<<Method for producing N-vinylformamide>>

The method for producing N-vinylformamide of the invention includes a step (hydroxylation reaction step) of producing N-($\alpha$-hydroxyethyl)formamide by the method for producing N-($\alpha$-hydroxyethyl)formamide of the invention, a step (alkoxylation reaction step) of producing N-($\alpha$-alkoxyethyl)formamide by reacting the N-($\alpha$-hydroxyethyl)formamide obtained with an alcohol in presence of an acid catalyst, and a step (thermal decomposition reaction step) of producing N-vinylformamide by thermally decomposing the N-($\alpha$-alkoxyethyl)formamide obtained.

[Hydroxylation reaction step]

As the step (1), the same step as the [hydroxylation reaction step] described above can be adopted.

[Alkoxylation reaction step]

This step is a step of obtaining N-($\alpha$-alkoxyethyl)formamide by reacting (alkoxylation reaction) the N-($\alpha$-hydroxyethyl)formamide obtained in the hydroxylation reaction step with an alcohol in presence of an acid catalyst.

In the alkoxylation reaction, the reaction mixture obtained by the hydroxylation reaction described above may be used or N-($\alpha$-hydroxyethyl)formamide may be isolated from the reaction mixture and used.

As the alcohol to be used in the alkoxylation reaction, a primary or secondary alcohol is used. Alcohols having from 1 to 8 carbon atoms are preferable and alcohols having from 1 to 4 carbon atoms are more preferable from the viewpoint of reactivity and handling property of N-($\alpha$-hydroxyethyl)formamide. Specific examples of the alcohol may include methanol, ethanol, n-propanol, n-butanol, isobutyl alcohol, n-pentanol, n-hexanol, n-heptanol, n-octanol, benzyl alcohol, 2-methoxyethanol, 2-ethoxyethanol, 2-propoxyethanol, 2-butoxyethanol, diethylene glycol monomethyl ether, ethylene glycol, propylene glycol, 1,4-butanediol, and diethylene glycol. Among these, a primary alcohol is preferable and methanol which has a low boiling point as a raw material and provides a product having a low boiling point is particularly preferable.

In order to increase the yield of product, it is preferable to use an excessive amount of alcohol, and specifically, a molar amount to be from 1.1 to 50 times the amount of N-($\alpha$-alkoxyethyl)formamide is preferable and a molar amount to be from 2.0 to 30 times the amount of N-($\alpha$-alkoxyethyl)formamide is more preferable.

Examples of the acid catalyst to be used in the alkoxylation reaction may include a mineral acid, an organic acid, an ion exchange resin exhibiting weak acidity or strong acidity, and a solid acid catalyst. Among these, a strongly acidic catalyst is preferable, and specific examples thereof may include sulfuric acid, hydrochloric acid, nitric acid, sulfamic acid, methanesulfonic acid, and crosslinked polystyrene sulfonic acid.

As the amount of the acid catalyst used, the total amount of the amount required to neutralize the basic catalyst contained in N-($\alpha$-hydroxyethyl)formamide and the amount required to advance the alkoxylation reaction is required. The amount of the acid catalyst used as the total amount is preferably from 0.001% to 10% by moles and more preferably from 0.1% to 5% by moles with respect to N-(a-hydroxyethyl)formamide.

The specific aspect of the alkoxylation reaction is not particularly limited, but for example, the alkoxylation reaction is easily accomplished by adding an acid catalyst to a mixture of N-($\alpha$-hydroxyethyl)formamide and an alcohol or bringing these into contact with each other. In addition, a method in which an acid catalyst is dissolved in an alcohol in advance to prepare a catalyst solution and the catalyst solution is then added to N-($\alpha$-hydroxyethyl)formamide may be used.

The reaction temperature is preferably from -10°C to 60°C, more preferably from 0°C to 40°C, and still more preferably from 5°C to 30°C from the viewpoint of the reactivity of the alkoxylation reaction and the stability of N-($\alpha$-hydroxyethyl)formamide.

After the completion of the alkoxylation reaction, the acid catalyst is usually neutralized with an alkali compound or it is filtered and separated in a case in which the acid catalyst is in the form of solid such as an ion exchange resin. Incidentally, the neutralization treatment itself is not an essential operation, but it is preferable to conduct the neutralization treatment from the viewpoint of minimizing the decomposition of N-(a-alkoxyethyl)formamide which is a product in the purification and recovery step since N-($\alpha$-alkoxyethyl)formamide is more stable under a neutral condition.

Incidentally, the basic catalyst in the N-($\alpha$-hydroxyethyl)formamide obtained in the hydroxylation reaction step reacts with sulfuric acid to form a sulfate such as sodium sulfate or potassium sulfate, for example, in the case of using sulfuric acid as the acid catalyst in the alkoxylation reaction. This sulfate hardly dissolves in the reaction mixture after the completion of the alkoxylation reaction and can be thus separated from the N-($\alpha$-alkoxyethyl)formamide by using a filter or the like.

It is preferable that the N-($\alpha$-alkoxyethyl)formamide obtained in the alkoxylation reaction step is purified by distillation to remove impurities and the like after the acid catalyst is neutralized or removed in the case of a solid acid.

[Thermal decomposition reaction step]

This step is a step of obtaining N-vinylformamide by thermally decomposing the N-($\alpha$-alkoxyethyl)formamide obtained in the alkoxylation reaction step.

In the thermal decomposition reaction, the N-($\alpha$-alkoxyethyl)formamide is evaporated in an evaporator or the like to obtain a raw material gas and this raw material gas is subjected to vapor phase thermal decomposition in a thermal decomposition reactor. Subsequently, the thermally decomposed gas obtained by vapor phase thermal decomposition is condensed in a condenser, and N-vinylformamide is thus obtained.

The temperature at which the N-($\alpha$-alkoxyethyl)formamide is evaporated is preferably from 80°C to 380°C.

In addition, the evaporation of N-($\alpha$-alkoxyethyl)formamide is preferably conducted under reduced pressure, and the pressure at that time is preferably from 0.4 to 80 kPa.

In the vapor phase thermal decomposition reaction, intramolecular elimination of alcohol occurs and N-vinylformamide is thus formed.

The temperature at the time of vapor phase thermal decomposition reaction is preferably from 300°C to 600°C.

The temperature at the time of condensation of the thermally decomposed gas is preferably 300°C or more and more preferably 350°C or more until immediately before condensation. In addition, the resultant is preferably a liquid at 80°C or less and more preferably a liquid at 50°C or less after condensation.

[Effect]

According to the method for producing N-vinylformamide of the invention, there is no quality problem even when the reaction product remains in the reaction system, the crystals remaining in the reactor are dispersed in the reaction liquid to be a seed crystal, and the product of the next reaction is precipitated without newly adding a seed crystal. Hence, it is possible to cut down the labor costs for washing the kettle, the cost for treating the waste washwater, and the cost of equipment and labor costs for adding the seed crystal. In addition, it is possible to produce N-vinylformamide at a high yield.

EXAMPLES

Hereinafter, the invention will be more specifically described with reference to Examples, but the invention is not limited thereto. Incidentally, "%" represents "% by mass" unless otherwise stated.

[Example 1]

By using the production apparatus illustrated in Fig. 1, N-($\alpha$-hydroxyethyl)formamide was produce in the following manner.

A production apparatus 1 illustrated in Fig. 1 is configured to be equipped with a catalyst storage tank 10 for storing a basic catalyst, a catalyst dissolution tank 20 provided downstream of the catalyst storage tank 10, a feeder 30 for supplying the basic catalyst stored in the catalyst storage tank 10 to the catalyst dissolution tank 20, and a hydroxylation reaction tank 40 provided downstream of the catalyst dissolution tank 20.

An air knocker 11 for imparting vibration to the catalyst storage tank 10 by hammering is attached to the bottom portion of the catalyst storage tank 10.

The feeder 30 and the catalyst dissolution tank 20 are connected with each other via a first supply pipe 31.

The catalyst dissolution tank 20 and the hydroxylation reaction tank 40 are connected with each other via a second supply pipe 21.

Potassium hydrogencarbonate was stored as a basic catalyst in the catalyst storage tank 10 illustrated in Fig. 1, and 95.2 kg of formamide was charged into the catalyst dissolution tank 20.

While hammering the lower portion of the catalyst storage tank 10 by using the air knocker 11 (Model SK-30 manufactured by SEISH1N ENTERPRISE Co., Ltd.), potassium hydrogencarbonate stored in the catalyst storage tank 10 was supplied to the feeder 30 by being dropped thereonto, 1.69 kg of potassium hydrogencarbonate was supplied to the catalyst dissolution tank 20 through the feeder 30 over 30 minutes to prepare a formamide solution of potassium hydrogencarbonate.

Separately, 384 kg of toluene for industrial use was charged into the hydroxylation reaction tank 40 which was made of stainless steel and equipped with a stirrer 42 and a temperature controller (not illustrated), the hydroxylation reaction tank 40 was purged with nitrogen gas, 107 kg of acetaldehyde was then added to the toluene, and the temperature was adjusted to 20°C.

The reaction tank 40 was not washed after the completion of the previous batch reaction, and crystals of N-($\alpha$-hydroxyethyl)formamide adhered to the upper wall surface of the reactor above the interface and the upper portion of the stirring blade shaft.

The stirrer was adjusted so that the stirring power of the reaction tank was 2.3 KW/m$^3$, and 20% of the formamide solution of potassium hydrogencarbonate in the catalyst dissolution tank 20 was added to the toluene solution of acetaldehyde in the hydroxylation reaction tank 40 over 15 minutes.

From the inlet temperature of cooling water of 19.0°C, the outlet temperature of 20.0°C, and the flow rate of cooling water of 4231 L in the hydroxylation reaction tank 40 immediately after the addition of 20% of formamide solution, the heat value by reaction in the hydroxylation reaction at this time was calculated to be approximately 10 kcal/mol.

Thereafter, the remaining amount of the formamide solution of potassium hydrogencarbonate was further added into the reaction tank over 3 hours, and the mixture was subjected to aging (hydroxylation reaction) for 1 hour to obtain a reaction slurry.

The reaction slurry obtained was filtered to separate toluene of a solvent. The gravity filtration time was 20 minutes. In addition, the average particle diameter N-(a-hydroxyethyl)formamide was approximately 1.0 mm as observed under a microscope.

A part of the solid component (reaction mixture) filtered was sampled and analyzed by liquid chromatography under the following conditions, and as a result, the reaction mixture contained N-($\alpha$-hydroxyethyl)formamide at 64.3%, formamide at 0.7%, acetaldehyde at 1.4%, and an aldol condensate of acetaldehyde at 0.2%. The percent conversion of formamide (abbreviated as the "FAM conversion" in Table 1) at this time was 97.9%. These results are presented in Table 1.

(Conditions for liquid chromatography analysis)

- Column: MCI-GEL-ODS 1HU (4.6 mm $\phi \times$ 250 mm).
- Flow rate: 1 mL/min.
- Eluent: 0.01 M-NaH$_2$PO$_3 \cdot$2H$_2$O.
- Sample injection volume: 20 $\mu$L of sample diluted with eluent by 1000 times.

[Example 2]

Crystals of N-($\alpha$-hydroxyethyl)formamide adhered to the interior of the hydroxylation reaction tank 40 after the completion of reaction in Example 1 in the same manner as at the start of Example 1. The amount of crystals adhered was approximately the same as that at the start of Example 1 by visual observation. The hydroxylation reaction was conducted by the same operation as in Example 1 except that the interior of the hydroxylation reaction tank 40 was not washed and the stirring power was adjusted to 1 KW/m$^3$. The results are presented in Table 1.

[Comparative Example 1]

The reaction was conducted in the same manner as in Example 2 except that the tank 40 was washed with water and dried before starting the reaction, and the reaction was started in a state in which crystals had not adhered to the wall, shaft, lid, and the like. To the toluene solution of acetaldehyde in the hydroxylation reaction tank 40, 20% of the formamide solution of potassium hydrogencarbonate in the catalyst dissolution tank 20 was added over 15 minutes, but the heat value was only about 60% of those in Examples 1 and 2, and crystallization was also not confirmed by visual observation. The liquid was sampled and analyzed, and the percent conversion of formamide was 66.8%. When 1 kg of seed crystal was charged into the reaction tank, heat generation rapidly occurred and crystallization proceeded. The dropwise addition of formamide solution was restarted as it was, and a FAM conversion of 97% was finally obtained.

[Table 1]

| | Crystal | Power | Heat generation | Residual FAM | OH form | Filtration time | Average particle diameter | FAM conversion |
|---|---|---|---|---|---|---|---|---|
| Example 1 | Presence of previous batch crystal | 2.3 KW/m$^3$ | About 10 kcal | 0.7% | 64.3% | 20 minutes | 1.0 mm | 97.9% |
| Example 2 | Presence of previous batch crystal | 1.0 KW/m$^3$ | About 10 kcal | 0.7% | 64.2% | 15 minutes | 1.2 mm | 97.9% |
| Comparative Example 1 | Washing of previous batch Absence of crystal remaining | 1.0 KW/m$^3$ | About 6 kcal | 9.7% | 38.6% | | | 66.8% |
| | Addition of seed crystal | | Rapid heat generation | | | | 1.2 mm | 97.00% |

The abbreviations in Table 1 are as follows.

- FAM: Formamide
- OH form: N-($\alpha$-hydroxyethyl)formamide

As is clear from the results in Table 1, the FAM conversion in the respective Examples was 97.9%. In Comparative Example in which the reaction was started in a state in which the reaction tank was washed and crystals of N-(a-hydroxyethyl)formamide did not remain in the tank, the FAM conversion reached 97.0% by adding a seed crystal in the middle of the operation.

INDUSTRIAL APPLICABILITY

According to the method for producing N-($\alpha$-hydroxyethyl)formamide of the invention, it is possible to produce N-($\alpha$-hydroxyethyl)formamide at a high yield. Moreover, it is possible to cut down the labor costs of washing and the cost for treating the waste washwater. In addition, it is possible to cut down the cost of equipment and labor costs for adding the seed crystal.

According to the method for producing N-vinylformamide of the invention, it is possible to produce N-vinylformamide at a high yield. Moreover, it is possible to cut down the labor costs of washing and the cost for treating the waste washwater. In addition, it is possible to cut down the cost of equipment and labor costs for adding the seed crystal.

EXPLANATIONS OF LETTERS OR NUMERALS

| 1 | PRODUCTION APPARATUS |
|---|---|
| 10 | CATALYST STORAGE TANK |
| 11 | AIR KNOCKER |
| 20 | CATALYST DISSOLUTION TANK |
| 21 | SECOND SUPPLY PIPE |
| 30 | FEEDER |
| 31 | FIRST SUPPLY PIPE |
| 40 | HYDROXYLATION REACTION TANK |
| 42 | STIRRER |

**Claims**

1. A method for producing N-($\alpha$-hydroxyethyl)formamide, comprising:

    reacting formamide with acetaldehyde in a solvent in presence of a basic catalyst in a reaction tank;
    precipitating N-($\alpha$-hydroxyethyl)formamide obtained as a crystal in the reaction tank; and
    recovering the crystal of N-($\alpha$-hydroxyethyl)formamide precipitated from the reaction tank, wherein
    an n+1-th reaction is conducted in a state in which the reaction solution is discharged from the reaction tank and at least a part of the crystals of N-($\alpha$-hydroxyethyl)formamide obtained by an n-th reaction remains in the reaction tank (where n is a natural number) when the production of N-($\alpha$-hydroxyethyl)formamide is conducted plural times in the same reaction tank.

2. The method for producing N-($\alpha$-hydroxyethyl)formamide according to claim 1, wherein an n+1-th reaction is conducted in a state in which the crystals of N-($\alpha$-hydroxyethyl)formamide obtained by an n-th reaction are present in an amount of 10 g or more and 200 kg or less per 1 $m^3$ of an inner volume of the reaction tank (where n is a natural number) when the production of N-($\alpha$-hydroxyethyl)formamide is conducted plural times in the same reaction tank.

3. The method for producing N-($\alpha$-hydroxyethyl)formamide according to claim 1 or 2, wherein an n+1-th reaction is conducted while stirring a reaction liquid in the reaction tank at a stirring power of from 0.1 to 10.0 kW/$m^3$.

4. The method for producing N-($\alpha$-hydroxyethyl)formamide according to claim 3, wherein the stirring power is from 0.4 to 6.0 kW/$m^3$.

5. The method for producing N-(α-hydroxyethyl)formamide according to any one of claims 1 to 4, wherein the solvent is at least one kind selected from the group consisting of an aliphatic hydrocarbon and an aromatic hydrocarbon.

6. The method for producing N-(α-hydroxyethyl)formamide according to any one of claims 1 to 5, wherein an average particle diameter of the crystals of N-(α-hydroxyethyl)formamide is from 0.3 to 2.0 mm.

7. A method for producing N-vinylformamide, comprising:

a step of producing N-(α-hydroxyethyl)formamide by the method for producing N-(α-hydroxyethyl)formamide according to any one of claims 1 to 6;
a step of producing N-(α-alkoxyethyl)formamide by reacting the N-(α-hydroxyethyl)formamide obtained with an alcohol in presence of an acid catalyst; and
a step of producing N-vinylformamide by thermally decomposing the N-(α-alkoxyethyl)formamide obtained.

**Patentansprüche**

1. Verfahren zur Herstellung von N-(a-Hydroxyethyl)formamid, umfassend:

Umsetzen von Formamid mit Acetaldehyd in einem Lösungsmittel in Gegenwart eines basischen Katalysators in einem Reaktionstank;
Ausfällen von N-(α-Hydroxyethyl)formamid, das als Kristall erhalten wird, in dem Reaktionstank; und
Gewinnung des Kristalls von ausgefälltem N-(a-Hydroxyethyl)formamid aus dem Reaktionstank, wobei eine n+1-te Reaktion in einem Zustand durchgeführt wird, in dem die Reaktionslösung aus dem Reaktionstank abgelassen wird und mindestens ein Teil der Kristalle von N-(α-Hydroxyethyl)formamid, die durch eine n-te Reaktion erhalten wurden, in dem Reaktionstank verbleibt (wobei n eine natürliche Zahl ist), wenn die Herstellung von N-(α-Hydroxyethyl)formamid mehrmals in demselben Reaktionstank durchgeführt wird.

2. Verfahren zur Herstellung von N-(α-Hydroxyethyl)formamid gemäß Anspruch 1, wobei eine n+1-te Reaktion in einem Zustand durchgeführt wird, in dem die Kristalle von N-(α-Hydroxyethyl)formamid, die durch eine n-te Reaktion erhalten wurden, in einer Menge von 10 g oder mehr und 200 kg oder weniger pro 1 m$^3$ des inneren Volumens des Reaktionstanks vorliegen (wobei n eine natürliche Zahl ist), wenn die Herstellung von N-(a-Hydroxyethyl)formamid mehrmals in demselben Reaktionstank durchgeführt wird.

3. Verfahren zur Herstellung von N-(α-Hydroxyethyl)formamid gemäß Anspruch 1 oder 2, wobei eine n+1-te Reaktion unter Rühren einer Reaktionsflüssigkeit im Reaktionstank bei einer Rührleistung von 0,1 bis 10,0 kW/m$^3$ durchgeführt wird.

4. Verfahren zur Herstellung von N-(α-Hydroxyethyl)formamid gemäß Anspruch 3, wobei die Rührleistung von 0,4 bis 6,0 kW/m$^3$ beträgt.

5. Verfahren zur Herstellung von N-(α-Hydroxyethyl)formamid gemäß einem der Ansprüche 1 bis 4, wobei das Lösungsmittel mindestens eines ist, ausgewählt aus der Gruppe bestehend aus einem aliphatischen Kohlenwasserstoff und einem aromatischen Kohlenwasserstoff.

6. Verfahren zur Herstellung von N-(α-Hydroxyethyl)formamid gemäß einem der Ansprüche 1 bis 5, wobei ein durchschnittlicher Partikeldurchmesser der Kristalle von N-(α-Hydroxyethyl)formamid von 0,3 bis 2,0 mm beträgt.

7. Verfahren zur Herstellung von N-Vinylformamid, umfassend:

einen Schritt der Herstellung von N-(a-Hydroxyethyl)formamid nach dem Verfahren zur Herstellung von N-(α-Hydroxyethyl)formamid gemäß einem der Ansprüche 1 bis 6;
einen Schritt der Herstellung von N-(a-Alkoxyethyl)formamid durch Umsetzen des erhaltenen N-(a-Hydroxyethyl)formamids mit einem Alkohol in Gegenwart eines Säurekatalysators; und
einen Schritt der Herstellung von N-Vinylformamid durch thermische Zersetzung des erhaltenen N-(a-Alkoxyethyl)formamids.

**Revendications**

1. Procédé de production de N-($\alpha$-hydroxyéthyl)formamide, comprenant :

   la réaction du formamide avec de l'acétaldéhyde dans un solvant en présence d'un catalyseur basique dans une cuve de réaction ;
   la précipitation du N-($\alpha$-hydroxyéthyl)formamide obtenu sous forme de cristal dans la cuve de réaction ; et
   la récupération du cristal de N-(a-hydroxyéthyl)formamide précipité dans la cuve de réaction, dans lequel une n+1-ième réaction est effectuée dans un état dans lequel la solution réactionnelle est évacuée de la cuve de réaction et au moins une partie des cristaux de N-(a-hydroxyéthyl)formamide obtenus par une n-ième réaction reste dans la cuve de réaction (où n est un nombre naturel) quand la production du N-($\alpha$-hydroxyéthyl)-formamide est effectuée plusieurs fois dans la même cuve de réaction.

2. Procédé de production de N-($\alpha$-hydroxyéthyl)formamide selon la revendication 1, dans lequel une n+1-ième réaction est effectuée dans un état dans lequel les cristaux de N-(a-hydroxyéthyl)formamide obtenus par une n-ième réaction sont présents en une quantité de 10 g ou plus et de 200 kg ou moins par 1 $m^3$ d'un volume interne de la cuve de réaction (où n est un nombre naturel) quand la production de N-($\alpha$-hydroxyéthyl)formamide est effectuée plusieurs fois dans la même cuve de réaction.

3. Procédé de production de N-($\alpha$-hydroxyéthyl)formamide selon la revendication 1 ou 2, dans lequel une n+1-ième réaction est effectuée tout en agitant le liquide réactionnel dans la cuve de réaction à une puissance d'agitation de 0,1 à 10,0 $kW/m^3$.

4. Procédé de production de N-($\alpha$-hydroxyéthyl)formamide selon la revendication 3, dans lequel la puissance d'agitation est de 0,4 à 6,0 $kW/m^3$.

5. Procédé de production de N-($\alpha$-hydroxyéthyl)formamide selon l'une quelconque des revendications 1 à 4, dans lequel le solvant est au moins un type sélectionné dans le groupe consistant en un hydrocarbure aliphatique et un hydrocarbure aromatique.

6. Procédé de production de N-($\alpha$-hydroxyéthyl)formamide selon l'une quelconque des revendications 1 à 5, dans lequel un diamètre moyen de particule des cristaux de N-(a-hydroxyéthyl)formamide est de 0,3 à 2,0 mm.

7. Procédé de production de N-vinylformamide, comprenant :

   une étape de production de N-(a-hydroxyéthyl)formamide par le procédé de production de N-(a-hydroxyéthyl)formamide selon l'une quelconque des revendications 1 à 6 ;
   une étape de production de N-(a-alcoxyéthyl)formamide par réaction du N-(a-hydroxyéthyl)formamide obtenu avec un alcool en présence d'un catalyseur acide ; et
   une étape de production de N-vinylformamide par décomposition thermique du N-(a-alcoxyéthyl)formamide obtenu.

FIG. 1

**REFERENCES CITED IN THE DESCRIPTION**

**Patent documents cited in the description**

- JP 2017006763 A
- JP 6298713 A
- JP 2000191625 A
- JP 2007023284 A